(19) Europäisches Patentamt
European Patent Office
Office européen des brevets

(11) Veröffentlichungsnummer: **0 142 757**
A2

# (12) EUROPÄISCHE PATENTANMELDUNG

(21) Anmeldenummer: **84113234.3**

(22) Anmeldetag: **03.11.84**

(51) Int. Cl.[4]: **C 07 C 127/26**
C 07 C 157/14, C 07 C 129/12
C 07 D 295/20, C 07 D 265/30
A 01 N 47/42, A 01 N 47/44

(30) Priorität: **11.11.83 DE 3340770**

(43) Veröffentlichungstag der Anmeldung:
**29.05.85 Patentblatt 85/22**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Liebl, Rainer, Dr.**
**An der Weinleite 5b**
**D-8901 Todtenweis(DE)**

(72) Erfinder: **Handte, Reinhard, Dr.**
**Theilweg 23**
**D-8901 Gablingen(DE)**

(72) Erfinder: **Mildenberger, Hilmar, Dr.**
**Fasanenstrasse 24**
**D-6233 Kelkheim (Taunus)(DE)**

(72) Erfinder: **Bauer, Klaus, Dr.**
**Kolpingstrasse 7**
**D-6054 Rodgau(DE)**

(72) Erfinder: **Bieringer, Hermann, Dr.**
**Eichenweg 26**
**D-6239 Eppstein/Taunus(DE)**

(54) **Phenoxyalkanoylderivate, Verfahren zu ihrer Herstellung sowie ihre Verwendung im Pflanzenschutz.**

(57) Verbindungen der Formel I

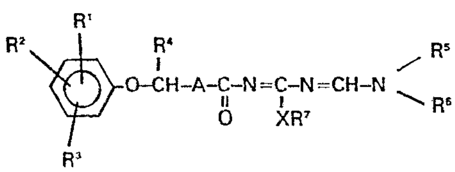

I,

worin bedeuten:

$R^1$, $R^2$, $R^3$ unabhängig voneinander Wasserstoff, F, Cl, Br oder ($C_1$–$C_4$)–Alkyl; $R^1$ außerdem $CF_3$, CN, $NO_2$ oder Alkoxycarbonyl; A eine direkte Bindung, $-CH_2-CH_2-$ oder $-CH=CH-$, X O,S oder N–$R^8$, $R^4$ H oder Alkyl, $R^5$ und $R^6$ Alkyl, $R^7$ Alkyl, (subst.) Benzyl und/oder (subst.) Phenethyl, $R^8$ H oder Alkyl oder zusammen mit $NR^7$ einen heterocyclischen Ring bedeuten, sind wirksame Herbizide und Antidots.

EP 0 142 757 A2

0142757

HOECHST AKTIENGESELLSCHAFT HOE 83/F 235 Dr.TG/mk

Phenoxyalkanoylderivate, Verfahren zu ihrer Herstellung sowie ihre Verwendung im Pflanzenschutz

Gegenstand der vorliegenden Erfindung sind neue Verbindungen der allgemeinen Formel I

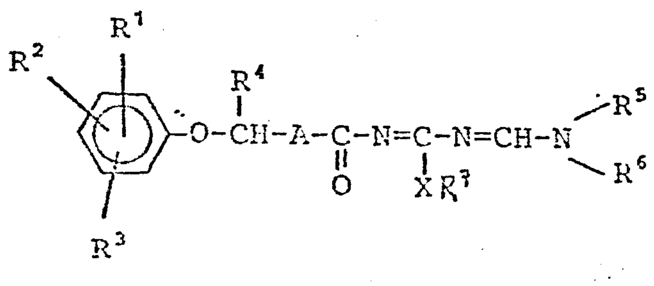

I,

worin bedeuten:

$R^1$, $R^2$, $R^3$ unabhängig voneinander Wasserstoff, F, Cl, Br oder $(C_1-C_4)$-Alkyl; $R^1$ außerdem $CF_3$, CN, $NO_2$ oder COO-$(C_1-C_4)$-Alkyl;

A eine direkte Bindung, $-CH_2-CH_2-$ oder $-CH=CH-$;

X O, S oder $NR^8$;

$R^4$ H oder $(C_1-C_4)$-Alkyl;

$R^5$, $R^6$ gleiche oder verschiedene $(C_1-C_4)$-Alkylreste;

$R^7$ $(C_1-C_4)$-Alkyl, Benzyl oder Phenylethyl, worin die Phenylreste ein- oder zweifach durch Halogen, $CF_3$, $NO_2$ oder $(C_1-C_4)$-Alkyl substituiert sein können;

$R^8$ H oder $(C_1-C_4)$-Alkyl;

sowie $R^7$ und $R^8$ zusammen mit dem Stickstoffatom einen Pyrrolidin-, Piperidin-, Morpholin- oder Piperazinring, die gegebenenfalls noch durch 1-2 $CH_3$-Gruppen substituiert sein können.

Bevorzugte Verbindungen der allgemeinen Formel I sind diejenigen, in denen

$R^1$, $R^2$, $R^3$ unabhängig voneinander Wasserstoff, Chlor, Brom und/oder Methyl bedeuten, A eine direkte Bindung darstellt, X Sauerstoff, $R^4$ Wasserstoff oder Methyl, $R^5$ und $R^6$ jeweils Methyl und $R^7$ $(C_1-C_4)$-Alkyl bedeuten.

Gegenstand der Erfindung ist weiterhin ein Verfahren zur Herstellung der Verbindungen I, das dadurch gekennzeichnet ist, daß man Verbindungen der allgemeinen Formel II

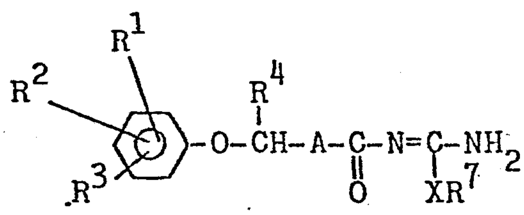

II

mit Dialkylformamid-dialkylacetalen der allgemeinen Formel III

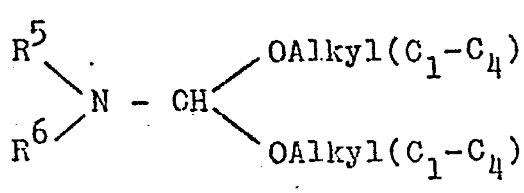

III

umsetzt.

Die Umsetzung wird in An- und Abwesenheit eines Verdünnungsmittels, jedoch in Gegenwart eines sauren Katalysators durchgeführt, wobei die Temperatur zwischen 20°C und 150°C liegen kann.

Bevorzugt wird ohne Lösungsmittel mit konz. Schwefelsäure als Katalysator bei Temperaturen von 80 - 100°C gearbeitet. Als Katalysatoren kommen außerdem z.B. Eisessig, Essigsäureanhydrid und p-Toluolsulfonsäure in Frage.

Die für die Herstellung benötigten Verbindungen der allgemeine Formel II weden nach bekannten Methoden aus Methylisoharnstoff-sulfat und den entsprechenden Carbonsäurechloriden analog US-PS 3 914 224 dargestellt.

Überraschenderweise wurde gefunden, daß Verbindungen der Formel I die Eigenschaften haben, einerseits gegen zahlreiche Unkräuter sehr gut herbizid zu sein und andererseits phytotoxische Nebenwirkungen von Pflanzenschutzmitteln, insbesondere von Herbiziden, beim Einsatz in Nutzpflanzenkulturen zu vermindern und auszuschalten.

Erfindungsgemäße Verbindungen bekämpfen also einerseits Unkräuter in verschiedenen landwirtschaftlchen Kulturen, d.h. sie verfügen über sehr gute herbizide Eigenschaften und werden von Kulturpflanzen wie Getreide, Mais, Reis usw. aber auch von breitblättrigen Kulturpflanzen toleriert, ohne daß Schäden beobachtet werden.

Andererseits können sie in niedrigen Konzentrationen in Verbindung mit anderen Herbiziden ausgebracht werden und sind dann in der Lage, schädliche Nebenwirkungen der letzteren zu antagonisieren d.h. völlig aufzuheben, ohne deren herbizide Wirksamkeit zu beeinträchtigen.

Hierdurch kann das Einsatzgebiet herkömmlicher Pflanzenschutzmittel ganz erheblich vergrößert werden. Solche Verbindungen, die die Eigenschaften besitzen Kulturpflanzen gegen phytotoxische Schäden durch Herbizide zu schützen, ohne die eigentliche herbizide Wirkung dieser Mittel zu beeinträchtigen, werden "Antidote" oder "Safener" genannt.

Herbizide, deren phytotoxische Nebenwirkungen mittels Verbindungen der Formel I herabgesetzt werden können, sind z.B. Carbamate, Thiolcarbamate, Halogenacetanilide, substituierte Phenoxy- und Phenoxyphenoxy-carbonsäureester sowie Pyridyloxy-,Benzoxazolyloxy- bzw. Benzthiazolyloxy-phenoxycarbonsäureester, Dimedonoximabkömmlinge, ferner Chinolyl-, Naphthyl- und Chinoxalyloxyphenoxycarbonsäureester.

Beispielsweise seien, ohne daß dadurch eine Beschränkung erfolgen soll, Herbizide aus folgenden Klassen genannt:

A) Herbizide vom Typ der Phenoxyphenoxycarbonsäure-($C_1$-$C_4$)alkyl- ($C_2$-$C_4$)alkenyl- und ($C_3$-$C_4$)alkinylester wie etwa

2-(4-(2,4-Dichlorphenoxy)-phenoxy)-propionsäuremethylester,
2-(4-(4-Brom-2-chlorphenoxy)-phenoxy)-propionsäuremethylester,
2-(4-(4-Trifluormethylphenoxy)-phenoxy)-propionsäuremethylester,
2-(4-(2-Chlor-4-trifluormethylphenoxy)-phenoxy)-propionsäuremethylester,
2-(4-(2,4-Dichlorbenzyl)-phenoxy)-propionsäuremethylester,
4-(4-(4-Trifluormethylphenoxy)-phenoxy)-pent-2-en-säureethylester,
2-(4-(3,5-Dichlorpyridyl-2-oxy)-phenoxy)-propionsäureethylester,
2-(4-(3,5-Dichlorpyridyl-2-oxy)-phenoxy)-propionsäurepropargylester,
2-(4-(6-Chlorbenzoxazol-2-yl-oxy)-phenoxy)-propionsäureethylester,
2-(4-(6-Chlorbenzthiazol-2-yl-oxy)-phenoxy)-propionsäureethylester,
2-(4-(3-Chlor-5-trifluormethyl-2-pyridyloxy)-phenoxy)-propionsäuremethylester,
2-(4-(5-Trifluormethyl-2-pyridyloxy)-phenoxy)-propionsäurebutylester,
2-(4-(6-Chlor-2-chinoxalinyloxy)-phenoxy)-propionsäureethylester

B) Chloracetanilid-Herbizide wie etwa

N-Methoxymethyl-2,6-diethyl-chloracetanilid,
N-(3'-Methoxyprop-2'-yl)-2-methyl-6-ethyl-chloracetanilid,
N-(3-Methyl-1,2,4-oxdiazol-5-ylmethyl)-chloressigsäure-2,6-dimethylanilid ...

C) Thiolcarbamate wie etwa

S-Ethyl-N,N-dipropylthiocarbamat oder
S-Ethyl-N,N-diisobutylthiocarbamat

D) Dimedon-Derivate wie etwa

2-(N-Ethoxybutyrimidoyl)-5-(2-ethylthiopropyl)-3-hydroxy-2-cyclohexen-1-on,
2-(N-Ethoxybutyrimidoyl)-5-(2-phenylthiopropyl)-3-hydroxy-2-cyclohexen-1-on oder
2-(1-Allyloxyiminobutyl)-4-methoxycarbonyl-5,5-dimethyl-3-oxocyclohexenol

Für die Anwendung können die Verbindungen der Formel I mit üblichen Formulierungshilfsmitteln zu Stäubemitteln, Spritzpulvern, Dispersionen, Emulsionskonzentraten usw. zubereitet werden, die den Wirkstoff in Konzentrationen von 2 - 80 % enthalten und entweder als solche angewendet werden (Stäubemittel, Pellets) oder vor der Anwendung in einem Lösungsmittel (Wasser) gelöst oder dispergiert werden.

Das Mengenverhältnis Antidot : Herbizid kann innerhalb weiter Grenzen zwischen 0,01 und 10 Teilen Antidot auf 1 Teil Herbizid schwanken. Die jeweils optimalen Mengen an Herbizid und Antidot sind abhängig vom Typ des verwendeten Herbizids bzw. Antidots sowie von der Art des zu behandelnden Pflanzenbestandes und lassen sich von Fall zu Fall durch entsprechende Versuche ermitteln.

Als Haupteinsatzgebiete für die erfindungsgemäßen Mittel kommen vor allem Kulturen von Getreide (Weizen, Roggen, Gerste, Hafer), Reis, Mais, Sorghum, aber auch Baumwolle, Zuckerrüben, Zuckerrohr, Sojabohne und andere in Frage.

...

Die erfindungsgemäßen Mittel können je nach ihren Eigenschaften zur Vorbehandlung des Saatgutes der Kulturpflanze (Beizung der Samen) oder vor der Saat in die Saatfurchen oder als Tankmischung vor oder nach dem Auflaufen der Pflanzen verwendet werden. Vorauflaufbehandlung schließt sowohl die Behandlung der Anbaufläche vor der Aussaat als auch die Behandlung der angesäten, aber noch nicht bewachsenen Anbauflächen ein.

Grundsätzlich kann das Antidot vor, nach oder gleichzeitig mit einem Herbizid angewendet werden, bevorzugt ist jedoch die gleichzeitige Anwendung in Form von Tankmischungen oder gegebenenfalls Fertigformulierungen.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium, dibutylnaphtalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt

...

werden. Bei flüssigen Wirkstoffen kann der Lösungsmittelanteil auch ganz oder teilweise entfallen. Als Emulgatoren können beispielsweise verwendet werden: Alkyl-arylsulfonsaure Calciumsalze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit fein verteilten, festen Stoffe, z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich.

Nachstehend seien einige Formulierungsbeispiele aufgeführt:

Ein Stäubemittel wird erhalten, indem man 10 Gewichtsteile Wirkstoff und 90 Gewichtsteile Talkum oder Inertstoff mischt und in einer Schlagmühle zerkleinert.

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat wird erhalten, indem man 20 Gewichtsteile Wirkstoff mit 6 Gewichtsteilen Alkylphenolpolyglykolether (Triton

...

X 207), 3 Gewichtsteilen Isotridecanolpolyglykolether (8 AeO) und 71 Gewichtsteilen paraffinischem Mineralöl (Siedebereich z. B. ca. 255 bis über 377 °C) mischt und in einer Reibkugelmühle auf eine Feinheit von unter 5 Mikron vermahlt.

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol als Emulgator.

...

## A. Herstellungsbeispiele

### Beispiel 1

N-(2,4-Dichlorphenoxy-acetyl)-N'-dimethylaminomethylen-O-methyl-isoharnstoff

Zu 28 g (0,1 mol) N-(2,4-Dichlorphenoxy-acetyl)-O-methyl-isoharnstoff (vgl. Beisp. 1A) und 16,2 g (0,11 mol) DMF-diethylacetal gibt man 1/2 ml konz. Schwefelsäure zu und erhitzt unter Rühren rasch auf 100 °C. Sobald die Lösung zu sieden beginnt, entfernt man die Heizung und läßt auf RT abkühlen. Die ausgefallenen Kristalle werden mit 50 ml Ether verrührt, abgesaugt und über Nacht getrocknet. Man erhält 24 g (72 % d. Th.) an N-(2,4-Dichlorphenoxy-acetyl)-N'-dimethylaminomethylen-O-methyl-isoharnstoff in Form farbloser Kristalle mit einem Schmelzpunkt von 105 - 106 °C.

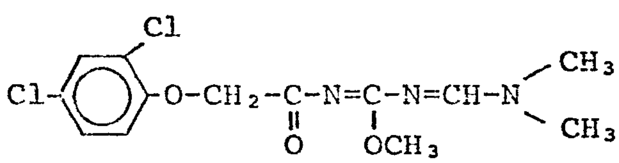

1

In analoger Weise werden die Verbindungen der Tabelle 1 hergestellt.

...

Tabelle 1

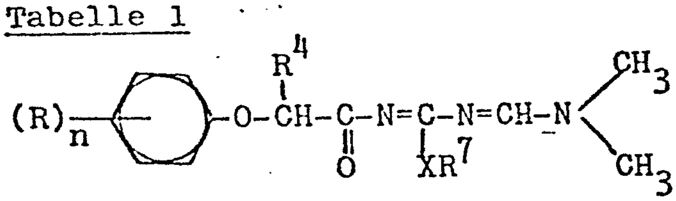

| Bei-spiel Nr. | $(R)_n$ | $R^4$ | $XR^7$ | Fp [°C] |
|---|---|---|---|---|
| 2 | $3,4-Cl_2$ | H | $OCH_3$ | 110-112 |
| 3 | H | H | $OCH_3$ | 72-73 |
| 4 | $3,4-Cl_2$ | $CH_3$ | $OCH_3$ | Oel |
| 5 | $2,5-Cl_2$ | H | $OCH_3$ | 144-147 |
| 6 | $4-Cl, 3-CH_3$ | H | $OCH_3$ | 117-119 |
| 7 | $4-Cl, 2-CH_3$ | H | $OCH_3$ | 89-90 |
| 8 | $2-i-C_3H_7, 5-CH_3$ | H | $OCH_3$ | Oel |
| 9 | $2,6-Cl_2, 4-CH_3$ | H | $OCH_3$ | 141-145 |
| 10 | $2,4,6-(CH_3)_3$ | H | $OCH_3$ | 130-131 |
| 11 | 4-Cl | H | $OCH_3$ | 92-94 |
| 12 | $2-CO_2Et$ | H | $OCH_3$ | Oel |
| 13 | $3-CO_2Et$ | H | $OCH_3$ | Oel |
| 14 | $2,3-(CH_3)_2$ | H | $OCH_3$ | 103-105 |
| 15 | $4-Cl, 3,5-(CH_3)_2$ | H | $OCH_3$ | 94-95 |
| 16 | $2-CH_3$ | H | $OCH_3$ | Oel |
| 17 | $3,5-Cl_2$ | H | $OCH_3$ | 142-144 |
| 18 | $2,4-Cl_2$ | H | $OC_2H_5$ | 106-107 |
| 19 | $4-Cl, 2-CH_3$ | H | $OC_2H_5$ | 72-73 |
| 20 | $2,3-(CH_3)_2$ | H | $OC_2H_5$ | 58-60 |
| 21 | $4-Cl, 3,5(CH_3)_2$ | H | $OC_2H_5$ | 82-84 |
| 22 | $3,4-Cl_2$ | H | $OC_2H_5$ | 104-106 |
| 23 | $2,4-Cl_2$ | H | $SCH_3$ | 104-105 |
| 24 | 4-Cl | H | $SCH_3$ | 98-100 |
| 25 | $4-Cl, 3,5-(CH_3)_2$ | H | $OCH_3$ | 88-92 |
| 26 | $4-CF_3$ | H | $OCH_3$ | |
| 27 | 4-CN | H | $OCH_3$ | |
| 28 | $3-NO_2$ | H | $OCH_3$ | |
| 29 | $4-COOC_2H_5$ | H | $OC_2H_5$ | |

0142757

Beispiel 30

1-(2,4-Dichlorphenoxy-acetyl)-3-dimethylaminomethylen-4-tetramethylen-quanidin

8,1 g (0,055 mol) Dimethylformamid-diethylacetal und 15,8 g (0,5 mol) 1-(2,4-Dichlorphenoxy-acetyl)-3-tetramethylen-guanidin werden mit 0,3 ml konz. Schwefelsäure 5 min auf 100°C erwärmt. Man destilliert bei 60°C/0,01 bar die flüchtigen Bestandteile ab. Es verbleiben 16 g (86 % d. Th.) 1-(2,4-Dichlorphenoxy-acetyl)-3-dimethylaminomethylen-4-tetramethylen-guanidin in Form eines zähen, gelben Öles.

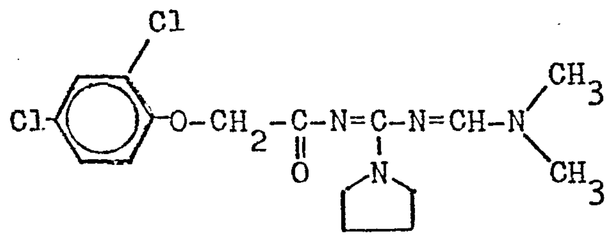

In analoger Weise erhält man:

Tabelle 2

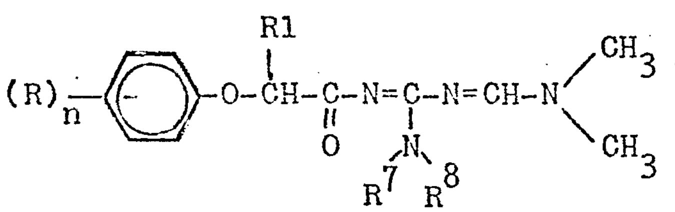

| Bei-spiel Nr. | $(R)_n$ | $R^1$ | $R^7$ | $R^8$ | Fp [°C] |
|---|---|---|---|---|---|
| 31 | 2,4-$Cl_2$ | H | 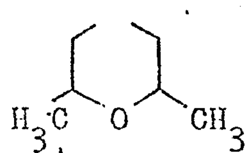 | | Oel |
| 32 | 2,4-$Cl_2$ | H | H | $-CH_2CH_2-OCH_3$ | Oel |

Beispiel 1 A (Herstellung der Ausgangsstoffe II)

N-(2,4-Dichlorphenoxy-acetyl)-O-methyl-isoharnstoff

13,5 (0,11 mol) O-Methyl-isoharnstoffsulfat werden in 50 ml Wasser und 100 ml Methylenchlorid gelöst. Bei 0°C tropft man unter kräftigem Rühren gleichzeitig aus 2 getrennten Gefäßen 16 g 50%ige wäßrige Natronlauge (0,2 mol) und 24 g (0,1 mol) 2,4-Dichlorphenoxyessigsäurechlorid zu. Man rührt 1 h bei 0°C und 3 h bei 40°C, trennt die organische Phase ab, wäscht 2mal mit 100 ml Wasser und trocknet über Natriumsulfat.

Nach dem Abdestillieren des Lösungsmittels verbleiben 22 g (79 % d. Th.) N-(2,4-Dichlorphenoxy-acetyl)-O-methyl-isoharnstoff in Form farbloser Kristalle vom Smp. 104°C.

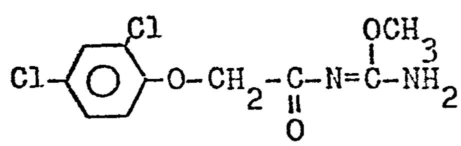

In analoger Weise wurden die anderen Verbindungen der Formel II erhalten.

B. Biologische Beispiele

I Safener-Wirkung

Weizen wurde im Gewächshaus in Töpfen von 9 cm ∅ bis zum 5-Blattstadium herangezogen und anschließend mit einem Herbizid und einer erfindungsgemäßen Verbindung tropfnaß gespritzt. 3 Wochen nach der Behandlung wurden die Pflanzena uf jede Art von Schädigung durch die Präparate bonitiert, wobei insbesondere das

0142757

Ausmaß der anhaltenden Wachstumshemmung berachtet wurde.

Die Ergebnisse aus Tabelle 3 veranschaulichen, daß die erfindungsgemäßen Verbindungen starke Herbizidschäden sehr effektiv reduzieren können.

Mischungen aus Herbiziden und erfindungsgemäßen Verbindungen eignen sich also zur selektiven Unkrautbekämpfung in Getreide.

Tabelle 3

Antidot-Wirkung der erfindungsgemäßen Verbindungen in Verbindung mit dem Herbizid Fenoxaprop-ethyl

| Verbindung | Dosis kg AS/ha | % Schädigung an Weizen |
|---|---|---|
| Herbizid F* | 2,0 | 70 |
| F + Bsp. 1 | 2,0 + 2,5 | 30 |
| F + Bsp. 2 | 2,0 + 2,5 | 20 |
| F + Bsp. 6 | 2,0 + 2,5 | 30 |
| F + Bsp. 18 | 2,0 + 2,5 | 30 |

*(F = Fenoxaprop-ethyl = 2-[4-(6-Chlor-2-benzoxazolyl-oxy)-phenoxy]-propionsäureethylester)

Die Safener-Wirkung weiterer Verbindungen zeigte ganz ähnliche Ausmaße bei Aufwandmengen von 2,5 kg AS/ha.

Die herbizide Wirksamkeit von Fenoxaprop-ethyl wurde durch die Zugabe der erfindungsgemäßen Safener nicht beeinträchtigt; sie betrug bei den angegebenen Aufwandmengen von z. B. 2,0 kg AS durchwegs immer 100 % z. B. bei Alopecurus myosuroides, bei Avena fatua und bei Hirsen wie Echinochloa- und Setaria-Arten.

0142757

Biologisches Beispiel 2

Weizen, Gerste und 2 wichtige Schadgräser Avena fatua und Alopecurus myosuroides wurden ausgesät und unter Gewächshausbedingungen bis zu 4-Blattstadium angezogen. Sodann wurden die erfindungsgemäßen Verbindungen und ein Herbizid in verschiedenen Dosierungen auf die Versuchspflanzen gesprüht. Nach 4 weiteren Wochen Standzeit im Gewächshaus wurden die Pflanzen auf jede Art von Hemmung oder Schädigung im Vergleich zu unbehandelten Kontrollpflanzen bonitiert. Die Ergebnisse (Tabelle 2) veranschaulichen, daß die erfindungsgemäße Verbindung herbizide Schadwirkungen an Weizen und Gerste sehr stark reduziert bzw. vollständig ausschaltet, ohne die herbizide Wirksamkeit gegen die Schadgräser Avena und Alopecurus zu beeinträchtigen.

Tabelle 2

Safener-Wirkung im Nachauflaufverfahren an Gerste und Weizen. Schädigung in %, im Vergleich zu unbehandeltenen Kontrollpflanzen.

| Verbindung | Dosis (kg AS/ha) | HV | TA | AVF | ALM |
|---|---|---|---|---|---|
| | 1,6 + 0,125 | - | 23 | | |
| | 0,8 + 0,125 | - | 10 | | |
| F + Bsp. 1 | 0,4 + 0,125 | 60 | 0 | - | 100 |
| | 0,2 + 0,125 | 23 | 0 | 99 | 97 |
| | 0,1 + 0,125 | 3 | | 99 | 97 |
| | 0,05 + 0,125 | 0 | | 97 | 95 |
| | 1,6 + 0,5 | | 0 | | |
| | 0,8 + 0,5 | | 0 | | |
| | 0,4 + 0,5 | 15 | 0 | | 100 |
| F + Bsp.1 | 0,2 + 0,5 | 0 | 0 | 99 | 99 |
| | 0,1 + 0,5 | 0 | | 99 | 99 |
| | 0,05 + 0,5 | 0 | | 96 | 97 |

Fortsetzung Tabelle 2

| Verbindung | Dosis (kg AS/ha) | HV | TA | AVF | ALM |
|---|---|---|---|---|---|
| F | 1,6 | - | 70 | | |
| | 0,8 | - | 55 | | |
| | 0,4 | 85 | 45 | | 99 |
| | 0,2 | 75 | 20 | 99 | 97 |
| | 0,1 | 65 | - | 99 | 95 |
| | 0,05 | 50 | - | 93 | 88 |
| Bsp. 1 | 0,5 | 0 | 0 | 0 | 0 |

HV = Gerste
TA = Weizen
AVF = Avena fatua
ALM = Alopecurus myosuroides

## II Herbizide Wirkung

In höheren Aufwandmengen zeigen die erfindungsgemäßen Verbindungen gute herbizide Eigenschaften gegen dikotyle und teilweise, besonders im Vorlauflaufverfahren, auch gegen monokotyle Unkräuter. Sie sind dahr zur selektiven Unkrautbekämpfung in landwirtschaftlichen Kulturen geeignet, wobei ihr Einsatz sowohl im Vorlauflaufverfahren wie auch im Nachauflaufverfahren möglich ist, und zwar besonders in Getreidekulturen in breitblättrigen Kulturen wie Sojabohne, Baumwolle oder Zuckerrüben.

Biologisches Beispiel 3

Breitblättrige und grasartige Unkräuter wurden in Plastiktöpfen von 9 cm Ø auf Lehmerde ausgesät und sowohl im Vorauflaufverfahren wie im Nachauflaufverfahren mit den erfindungsgemäßen Verbindungen (Aufwandmenge jeweils 2,4 kg a. i./ha) behandelt. 4 Wochen später wurde die herbizide Wirksamkeit durch visuelle Bonitur geschätzt.

Die Ergebnisse (Tabelle 3) zeigen, daß die erfindungsgemäßen Verbindungen ein weites Spektrum von Unkräutern im Vor- und Nachauflaufverfahren bekämpfen.

Tabelle 3

Herbizide Wirksamkeit (Schädigung in %)

| Beispiel Nr. | Vorauflauf | | | | Nachauflauf | | |
|---|---|---|---|---|---|---|---|
| | SIA | STM | CRS | LOM | SIA | AMR | STM |
| 1 | 5 | 5 | 5 | 5 | 5 | 5 | 5 |
| 3 | 5 | 5 | 3 | 4 | 5 | 5 | 5 |
| 2 | 5 | 5 | 5 | 5 | 5 | 5 | 5 |
| 5 | 5 | 5 | 5 | 5 | 5 | 5 | 5 |
| 6 | 5 | 5 | 5 | 5 | 5 | 5 | 4 |
| 7 | 5 | 5 | 5 | 5 | 5 | 5 | 5 |
| 8 | - | 3 | - | 3 | 3 | - | 4 |
| 23 | 5 | 5 | 5 | 5 | 5 | 5 | 5 |
| 18 | 5 | 3 | 5 | 5 | 3 | 5 | 5 |
| 19 | 5 | 5 | 5 | 5 | 5 | 5 | 5 |
| 20 | 5 | 5 | 5 | 4 | 3 | - | 4 |
| 10 | 3 | 3 | 3 | 2 | 3 | 4 | 3 |

...

SIA = Sinapis arvensis
STM = Stellaria media
CRS = Chrysanthemum segetum
LOM = Lolium multiflorum
AMR = Amaranthus retroflexus

Boniturschlüssel:
1 = 0 - 20 % Wirkung
2 = 20 - 40 % Wirkung
3 = 40 - 60 % Wirkung
4 = 60 - 80 % Wirkung
5 = 80 - 100 % Wirkung

...

PATENTANSPRÜCHE:

1. Verbindungen der allgemeinen Formel I

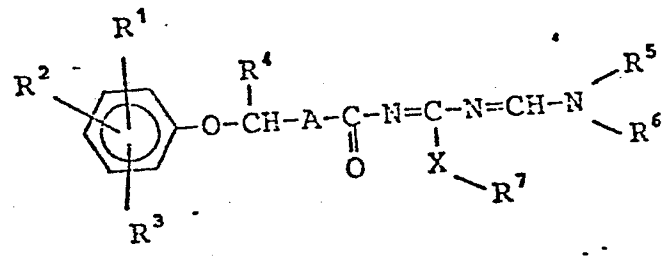

worin bedeuten:

$R^1$, $R^2$, $R^3$ unabhängig voneinander Wasserstoff, F, Cl, Br oder $(C_1-C_4)$-Alkyl; $R^1$ außerdem $CF_3$, CN, $NO_2$ oder $COO(C_1-C_4)$-Alkyl

A eine direkte Bindung, $-CH_2-CH_2-$ oder $-CH=CH-$;

X O, S oder $NR^8$;

$R^4$ H oder $(C_1-C_4)$-Alkyl;

$R^5$, $R^6$ gleiche oder verschiedene $(C_1-C_4)$-Alkylreste;

$R^7$ $(C_1-C_4)$-Alkyl, Benzyl oder Phenylethyl, worin die Phenylreste ein- oder zweifach durch Halogen, $CF_3$, $NO_2$ oder $(C_1-C_4)$-Alkyl substituiert sein können,

$R^8$ H oder $(C_1-C_4)$-Alkyl

 

sowie $R^7$ und $R^8$ zusammen mit dem Stickstoffatom einen Pyrrolidin-, Piperidin-, Morpholin- oder Pipera inring, die gegebenenfalls noch durch 1-2 $CH_3$-Gruppen substituiert sein können.

2. Verfahren zur Herstellung der Verbindungen gemäß Anspruch 1, dadurch gekennzeichnet, daß man Verbindungen der allgemeinen Formel

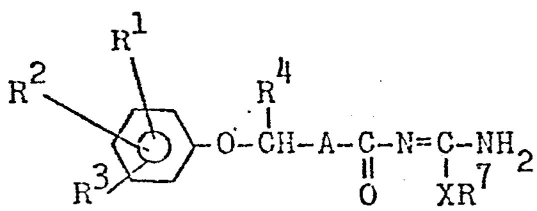

mit Verbindungen der allgemeinen Formel

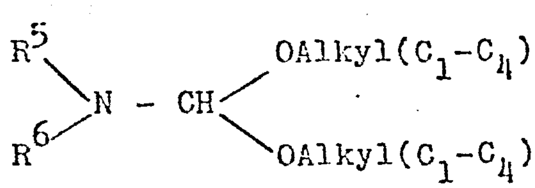

umsetzt.

3. Herbizide bzw. antidotwirksame Mittel, gekennzeichnet durch einen Gehalt an einer Verbindung gemäß Anspruch 1,

4. Verwendung von Verbindungen der Formel I als Herbizide bzw. Antidots (Safener)

5. Verfahren zum Schutz von Kulturpflanzen gegen phytotoxische Nebenwirkungen von Herbiziden, dadurch gekennzeichnet, daß man die Pflanzen, Pflanzenteile oder Böden mit einer wirksamen Menge einer Verbindung gemäß Anspruch 1 vor, nach oder gleichzeitig mit einem Herbizid behandelt.

6. Verfahren gemäß Anspruch 5, dadurch gekennzeichnet, daß das Herbizid ein Wirkstoff aus der Gruppe der Phenoxycarbonsäureester, Chloracetanilide, Thiolcarbamate oder Dimedon-Derivate ist.

7. Verfahren nach Anspruch 5, dadurch gekennzeichnet, daß das Herbizid aus der Gruppe der gräserwirksamen Phenoxycarbonsäureester stammt.

8. Verfahren gemäß Anspruch 5, dadurch gekennzeichnet, daß das Mischungsverhältnis von Herbizid : Antidot im Bereich 1 : 0,01 und 1 : 10 liegt.

9. Verfahren zur Bekämpfung von Schadpflanzen, dadurch gekennzeichnet, daß man auf die zu behandelnden Aufbauflächen eine herbizid wirksame Menge einer Verbindung gemäß Anspruch 1 aufbringt.

0142757

Patentansprüche für Österreich:

1. Verfahren zur Herstellung der Verbindungen der allgemeinen Formel I

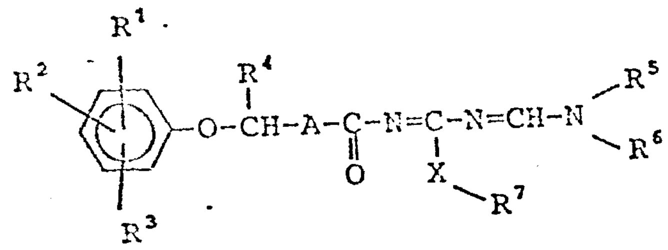

worin bedeuten:

$R^1$, $R^2$, $R^3$ unabhängig voneinander Wasserstoff, F, Cl, Br oder $(C_1-C_4)$-Alkyl; $R^1$ außerdem $CF_3$, CN, $NO_2$ oder $COO(C_1-C_4)$-Alkyl

A eine direkte Bindung, $-CH_2-CH_2-$ oder $-CH=CH-$;

X O, S oder $NR^8$;

$R^4$ H oder $(C_1-C_4)$-Alkyl;

$R^5$, $R^6$ gleiche oder verschiedene $(C_1-C_4)$-Alkylreste;

$R^7$ $(C_1-C_4)$-Alkyl, Benzyl oder Phenylethyl, worin die Phenylreste ein- oder zweifach durch Halogen, $CF_3$, $NO_2$ oder $(C_1-C_4)$-Alkyl substituiert sein können,

$R^8$ H oder $(C_1-C_4)$-Alkyl;

sowie $R^7$ und $R^8$ zusammen mit dem Stickstoffatom einen Pyrrolidin-, Piperidin-, Morpholin- oder Piperacinring, die gegebenenfalls noch durch 1-2 $CH_3$-Gruppen substitu-

iert sein können, bei dem man Verbindungen der allgemeinen Formel II

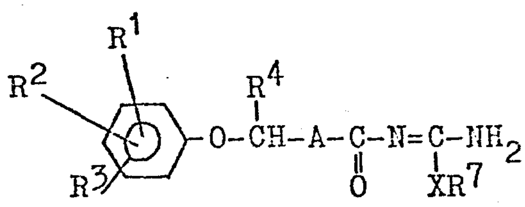

mit Verbindungen der allgemeinen Formel III

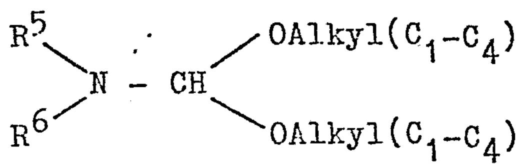

umsetzt.

2. Herbizide bzw. antidotwirksame Mittel, gekennzeichnet durch einen Gehalt an einer Verbindung der allgemeinen Formel I von Anspruch 1.

3. Verwendung von Verbindungen der allgemeinen Formel I von Anspruch 1 als Herbizide bzw. Antidots (Safener).

4. Verfahren zum Schutz von Kulturpflanzen gegen phytotoxische Nebenwirkungen von Herbiziden, dadurch gekennzeichnet, daß man die Pflanzen, Pflanzenteile oder Böden mit einer wirksamen Menge einer Verbindung der allgemeinen Formel I nach Anspruch 1 vor, nach oder gleichzeitig mit einem Herbizid behandelt.

5. Verfahren gemäß Anspruch 4, dadurch gekennzeichnet, daß das Herbizid ein Wirkstoff aus der Gruppe der

Phenoxycarbonsäureester, Chloracetanilide, Thiolcarbamate oder Dimedon-Derivate ist.

6. Verfahren nach Anspruch 4, dadurch gekennzeichnet, daß das Herbizid aus der Gruppe der gräserwirksamen Phenoxycarbonsäureester stammt.

7. Verfahren gemäß Anspruch 4, dadurch gekennzeichnet, daß das Mischungsverhältnis von Herbizid : Antidot im Bereich 1 : 0,01 und 1 : 10 liegt.

8. Verfahren zur Bekämpfung von Schadpflanzen, dadurch gekennzeichnet, daß man auf die zu behandelnden Aufbauflächen eine herbizid wirksame Menge einer Verbindung der allgemeinen Formel I nach Anspruch 1 aufbringt.